# EUROPÄISCHE PATENTSCHRIFT

(11) **EP 4 514 432 B1**
(45) Veröffentlichungstag und Bekanntmachung des Hinweises auf die Patenterteilung: **18.03.2026**
(21) Anmeldenummer: 23718248.0
(22) Anmeldetag: 06.04.2023
(51) Int. Cl.: A61M 11/00, A61M 15/00, A61M 15/08, B05B 11/10, G16H 20/13, G16H 40/63

(54) **SPENDER UND AUSWERTEEINHEIT HIERFÜR**
DISPENSER AND EVALUATION UNIT THEREFOR
DISTRIBUTEUR ET UNITÉ D'ÉVALUATION POUR LEDIT DISTRIBUTEUR

(30) Priorität: 26.04.2022 EP 22170085
(43) Veröffentlichungstag der Anmeldung: 05.03.2025
(73) Patentinhaber: Aptar Radolfzell GmbH, 78315 Radolfzell (DE)
(72) Erfinder: KOHNLE, Jörg, 78056 Villingen-Schwenningen (DE); HELMLINGER, Michael, 78315 Radolfzell-Böhringen (DE)
(74) Vertreter: Witte, Weller und Partner Patentanwälte mbB Stuttgart
(86) Internationale Anmeldenummer: PCT/EP2023/059209
(87) Internationale Veröffentlichungsnummer: WO 2023/208568

(56) Entgegenhaltungen:
- EP-A1- 3 701 987
- EP-A1- 4 137 184
- EP-A1- 4 169 500
- WO-A1-2011/056889
- WO-A1-2014/004437
- WO-A1-2021/209320
- WO-A1-93/25841
- WO-A2-2015/193833
- DE-A1- 102019 217 937
- KR-B1- 101 994 297
- US-A1- 2017 246 406
- US-A1- 2020 330 719
- US-A1- 2020 345 588
- US-A1- 2020 405 579
- US-A1- 2021 090 703
- US-B2- 6 651 844
- US-B2- 8 342 172

## Beschreibung

Die Erfindung betrifft einen Spender zum Austrag pharmazeutischer oder kosmetischer Medien sowie insbesondere eine elektronische Auswerteeinheit für einen solchen Spender.

Ein gattungsgemäßer Spender dient dem Austrag von Medien, insbesondere von pharmazeutischen Flüssigkeiten. Es ist bekannt, solche Spender mit Auswerteeinheiten zu versehen, die über eine Sensorik die Nutzung des Spenders erfassen, beispielsweise um eine nachfolgende Prüfung zu gestatten, ob ein Medikament in richtiger Weise und in Übereinstimmung mit einem Medikationsplan eingenommen wird.

Bekannte Auswerteeinheiten sind in den jeweiligen Spender baulich integriert oder sind lösbar am Spender angebracht, so dass sie vom Spender gelöst und an einen anderen Spender gleichen Typs angebracht werden können.

Aufgrund vieler unterschiedlicher Spendertypen ist der Aufwand zurjeweiligen Ergänzung der Spender um Auswerteeinheiten erheblich.

Die Dokumente WO 2021/209320 A1, US 6 651 844 B2, EP 3 701 987 A1, US 2017/246406 A1 und EP 4 137 184 A1 offenbaren Spender mit Auswerteeinheiten.

### AUFGABE UND LÖSUNG

Aufgabe der Erfindung ist es, eine Auswerteeinheit für einen Spender zur Verfügung zu stellen, die es gestattet, mit Spendern unterschiedlicher Art verwendet zu werden und sicher an diesen unterschiedlichen Spendern zum Zwecke der Erfassung der Nutzung befestigt zu werden. Erfindungsgemäß wird ein Spender zum Austrag pharmazeutischer oder kosmetischer Medien vorgeschlagen, der über eine elektronische Auswerteeinheit verfügt. Die Erfindung betrifft darüber hinaus auch die Auswerteeinheit als solche.

Ein erfindungsgemäßer Spender weist ein Gehäuse auf, das die Außenkontur des Spenders bildet und mehrteilig ausgebildet sein kann. Das Gehäuse weist einen Speicherkörper auf, der den Medienspeicher und insbesondere einen Flüssigkeitsspeicher bildet, in dem das Medium, insbesondere eine pharmazeutische Flüssigkeit, vor dem Austrag gelagert ist.

Der Spender weist eine Austragöffnung auf, durch die Medium aus dem Medienspeicher in eine umgebende Atmosphäre abgegeben werden kann.

Bevorzugt ist der Spender zur Ausbringung von Flüssigkeit ausgebildet, insbesondere in Form eines Strahls, eines Sprühstrahls, oder in Form von Einzeltropfen. Stattdessen kann der Spender jedoch auch für andere Medien vorgesehen sein, so insbesondere zur Ausbringung von Pulver zum Zwecke der Inhalation.

Ist der Spender als Flüssigkeitsspender ausgebildet, so kann es sich insbesondere um einen Pumpspender handeln, der über eine manuell betätigbare Pumpeinrichtung verfügt, die Flüssigkeit aus dem Flüssigkeitsspeicher zur Austragöffnung fördert. Alternativ handelt es sich um einen Quetschflaschenspender, der einen elastisch verformbaren Speicherkörper aufweist, durch dessen manuelle Komprimierung Flüssigkeit ausgetragen werden kann.

Der Spender kann weiterhin als Inhalator ausgebildet sein, insbesondere als Spender mit einem zur Abgabe einer Dosis der Flüssigkeit niederdrückbarem Container zur Abgabe einer definierten Dosis des Medikaments. Gemäß einem weiteren Beispiel kann der Spender als Nasalspender ausgebildet sein, insbesondere mit einem langgestreckten Nasalapplikator, an dessen distalem Ende die Austragöffnung vorgesehen ist.

Die elektronische Auswerteeinheit ist zur Erfassung der Nutzung des Spenders ausgebildet. Sie weist hierfür elektrische bzw. elektronische Komponenten auf, insbesondere eine Batterie, einen Prozessor und mindestens einen Sensor. Über den Sensor werden Daten erfasst, die Rückschlüsse auf die Spendernutzung zulassen. Die Sensoren können dabei dafür ausgebildet sein, unmittelbar eine externe Kraftbeaufschlagung zu erfassen, wie sie beispielsweise beim Niederdrücken einer Betätigungshandhabe zur Durchführung einer Pumpbetätigung auftritt. Vorzugsweise ist jedoch mindestens ein Sensor vorgesehen, der berührungslos und ohne externe Kraftbeaufschlagung Daten erfassen kann, wobei es sich insbesondere um ein Mikrofon und/oder einen Bewegungssensor handeln kann. Diese Arten von Sensoren eigenen sich besonders gut dafür, an Spendern unterschiedlicher Art und Ausbringungsart verwendet zu werden, wobei eine spendertypspezifische Anpassung bezüglich der Hardware nicht erforderlich ist. Beispielsweise können die Ausgangsdaten eines Mikrofons ausgewertet werden, um die beim jeweiligen Spendertyp charakteristischen Geräusche während der Nutzung zu erkennen.

Die Auswerteeinheit umfasst vorzugsweise eine Ausgabeeinrichtung, insbesondere in Form mindestens einer LED, eines Displays, eines Vibrators und/oder eines Lautsprechers, um Signale an den Nutzer abzugeben, beispielsweise um die Information zu übermitteln, dass ein Austrag sensiert worden ist oder gemäß Medikationsplan ansteht.

Die Auswerteeinheit ist vorzugsweise dafür ausgebildet, zusammen mit einem externen Gerät wie insbesondere einem Smartphone verwendet zu werden. Zur Kommunikation mit dem externen Gerät weist die Auswerteeinheit vorzugsweise ein Funkmodul, insbesondere ein Bluetooth- und/oder WIFI-Funkmodul oder aber ein GSM-/3G-/4G- oder 5G-Funkmodel auf.

Die Auswerteeinheit ist zur lösbaren Anbringung am Gehäuse des Spenders, und zwar am genannten Speicherkörper des Gehäuses vorgesehen. Um eine sichere Anlage der Auswerteeinheit am Gehäuse zu ermöglichen, weist die elektronische Auswerteeinheit eine Anlageseite auf, die im angebrachten Zustand der Auswerteeinheit in Richtung des Gehäuses des Spenders weist, wobei an dieser Anlageseite mindestens zwei zueinander parallele Anlagerippen zur Anlage am Gehäuse vorgesehen sind. Vorzugsweise handelt es sich um genau zwei oder drei zueinander parallele Anlagerippen, wobei die einzelnen Anlagerippen auch unterbrochen sein können und aus zueinander fluchtenden Rippensegmenten bestehen kann. Die Rippen bilden erhabene Bereiche zur Anlage am Gehäuse. Die Rippen weisen vorzugsweise eine Länge zwischen 10 mm und 30 mm auf. Die Breite der Rippen beträgt vorzugsweise 5 mm oder weniger, insbesondere vorzugsweise 3 mm oder weniger. An ihrer zum Gehäuse weisenden Seite sind die Rippen vorzugsweise mit einer ausgerundeten Kante versehen.

Die Auswerteeinheit ist vorzugsweise zur lösbaren Anbringung an einer Mantelfläche des Speicherkörper des Gehäuses vorgesehen. Der Speicherkörper selbst weist vorzugsweise im Bereich der Mantelfläche eine zylindrische Form auf, insbesondere mit einem kreisförmigen oder einem elliptischen Querschnitt.

Die Auswerteinheit weist vorzugsweise ein Elektronikgehäuse auf, welches die Anlageseite bildet und innerhalb dessen die elektronischen Komponenten der Auswerteinheit angeordnet sind, insbesondere also ein Prozessor, ein Speicher und eine Batterie sowie je nach Typ der Auswertung einen oder mehrere Sensoren. Dieses Elektronikgehäuse umgibt das Gehäuse des Spenders und insbesondere den Speicherkörper nicht vollständig, sondern ist vorzugsweise rucksackähnlich an einer Seite des Speicherkörpers vorgesehen, so dass der Spender einschließlich der Auswerteeinheit hierdurch eine asymmetrische Formgebung erhält. Insbesondere vorzugsweise überdeckt das Elektronikgehäuse in Umfangsrichtung höchstens 50% der Mantelfläche des Speicherkörpers, vorzugsweise höchstens 40%.

Damit das so an einer Seite des Spenders an der Oberfläche anliegende Elektronikgehäuse hält, ist vorzugsweise mindestens ein Befestigungselement vorgesehen, welches am Elektronikgehäuse angebracht ist und mittels dessen das Elektronikgehäuse an der Mantelfläche befestigt ist. Das Befestigungselement bzw. die Befestigungselemente liegen insbesondere in Form von Haltearmen (in nicht beanspruchten Ausführungsformen) oder, erfindungsgemäß, in Form eines flexiblen Befestigungsbandes vor, wie im Weiteren noch beschrieben ist.

Die im montierten Zustand bestimmungsgemäß am Spendergehäuse anliegenden Anlagerippen sind vorzugsweise an einer Rückseite dieses Elektronikgehäuses der Auswerteeinheit vorgesehen. Die Anlagerippen befinden sich vorzugsweise bezogen auf die Umfangsrichtung des Speicherkörpers in einem Teilbereich der Mantelfläche von maximal 40% des Umfangs des Speicherkörpers, vorzugsweise in einem Teilbereich der Mantelfläche von maximal 25% des Umfangs des Speicherkörpers.

Die Anlagerippen erstrecken sich vorzugsweise rein vertikal, worunter die Haupterstreckungsrichtung bezogen auf eine Zylinderform des Speicherkörpers 18 verstanden wird.

Zwischen den mindestens zwei Anlagerippen ist ein zurückgesetzter Zwischenbereich vorgesehen, der die Anlagerippen voneinander trennt. In diesem Zwischenbereich ist die Anlageseite der Auswerteeinheit derart zurückgesetzt, so dass sie nicht am Gehäuse des Spenders anliegt. Der Kontakt zwischen der Anlageseite und dem Gehäuse des Spenders beschränkt sich demnach auf die mindestens zwei Anlagerippen.

Eine Auswerteeinheit mit solchen Anlagerippen gestattet eine sichere Anlage am Gehäuse des Spenders, da die Andruckkraft in definierten Anlagebereichen stattfindet. Zudem gestatten die Anlagerippen die Anlage an Gehäusen unterschiedliche Maße und insbesondere Speicherkörper mit unterschiedlich gewölbter Außenkontur.

Die Anlagenrippen führen zu einem festen Halt der Auswerteinheit am Gehäuse des Spenders und insbesondere an dessen Speicherkörper. Bestimmungsgemäß bleiben die Anlagerippen zur Mantelfläche des Speicherkörpers ortsfest, wenn die Auswerteinheit angebracht ist. Relativbeweglichkeit im Betrieb ist nicht gewünscht.

Welche Krümmungsradien solcher Außenkonturen jeweils abgedeckt werden, hängt von der Geometrie der Anlageseite ab. Bevorzugt ist eine Gestaltung, bei der die Anlagerippen einen Abstand voneinander von mindestens 8 mm aufweisen, wobei sich die Anlagerippen vorzugsweise um mindestens 1 mm, vorzugsweise um mindestens 1,5 mm über die Anlagefläche in diesem Zwischenbereich erheben. Zwei Anlagerippen, zwischen denen ein Zwischenbereich von 8 mm vorgesehen ist, der 1 mm zurückgesetzt ist, können an Gehäuseabschnitten mit einem Krümmungsradius größer 10 mm angebracht werden, ohne dass der Gehäuseabschnitt abseits der Anlagerippen in Kontakt mit der Anlageseite gelangt. Mit einem Zwischenbereich von 8 mm Weite, der 1,5 mm zurückgesetzt ist, ist die Anbringung bis zu einem Krümmungsradius von ca. 6 mm möglich.

Die Anlagerippen weisen vorzugsweise einen Abstand von maximal 20 mm auf und weisen erfindungsgemäß bezogen auf den Durchmesser des Speicherkörpers einen demgegenüber geringeren Abstand auf, der maximal 80% des Durchmessers des Speicherkörpers beträgt.

Eine oder mehrere der Anlagerippen können an einem elastischen Ausleger angebracht sein, insbesondere an einem Ausleger in Form einer an drei Seiten freigeschnittenen Kunststoffzunge, die ein federndes Verhalten orthogonal zur Anlageseite ermöglicht. Es kann auch vorgesehen sein, dass eine oder mehrere, nicht aber alle Anlagerippen derartig auslenkbar sind, während mindestens eine Anlagerippe fest und nicht auslenkbar an der Anlageseite der Auswerteeinheit vorgesehen ist. Eine mögliche Konstellation sieht beispielsweise drei Anlagerippen vor, von denen eine oder zwei an jeweils einem elastischen Ausleger angebracht sind, während die dritte Anlagerippe ortsfest an der Anlageseite vorgesehen ist. Auch eine Rippengestaltung mit zwei Anlagerippen, die beide auslenkbar sind, kann zweckmäßig sein.

Im Falle einer Anlagerippe, die aus mehreren zueinander fluchtenden Rippensegmenten besteht, kann es vorteilhaft sein, wenn diese Rippensegmente an separaten elastischen Auslegern vorgesehen sind.

Die Anlagerippen als solche reichen nicht aus, um die Auswerteeinheit am Gehäuse des Spenders zu befestigen, sondern sie bewirken lediglich das sichere Anliegen der Auswerteeinheit an der Außenseite des Gehäuses.

Zum Zwecke der dortigen Befestigung sind unterschiedliche Konzepte möglich.

Erfindungsgemäß weist die elektronische Auswerteeinheit ein formflexibles Befestigungsband auf, welches an der Auswerteeinheit angebracht ist und das Gehäuse umgibt, und zwar den Speicherkörper. Mittels dieses Befestigungsbandes wird die Auswerteeinheit an das Gehäuse angedrückt. Zusammen mit den Anlagerippen lässt sich so ein sicherer Halt erzielen.

Das Befestigungsband kann als elastisches Band oder auch als weitgehend unelastisches Band ausgestaltet sein. Insbesondere bei Verwendung eines unelastischen Bandes ist es zweckmäßig, mindestens eine Anlagerippe an einem elastisch auslenkbaren Ausleger vorzusehen.

Ist das Befestigungsband elastisch, so kann es ohne Längenanpassung für Gehäuse und insbesondere Speicherkörper unterschiedlicher Umfänge verwendet werden.

Vorzugsweise ist das Befestigungsband aus einem anderen Material als das Elektronikgehäuse ausgebildet und als separates Element hergestellt und anschließend am Elektronikgehäuse angebracht.

Das Befestigungsband kann auf verschiedene Weisen an dem Elektronikgehäuse der Auswerteeinheit befestigt sein. Eine bevorzugte Gestaltung sieht vor, dass das Befestigungsband umlaufend geschlossen wird und zweisträngig um das Gehäuse herumgelegt wird, wobei die beiden jeweilig endseitigen Schlaufen am Gehäuse befestigt sind oder werden. Durch die Schlaufen ist die Befestigung am Gehäuse der Auswerteeinheit einfach möglich. Ein Befestigungsband, das derartig zweisträngig um das Gehäuse des Spenders herumgelegt wird, weist vorzugsweise einen kreisrunden Querschnitt auf und kann durch einen einfachen O-Ring gebildet werden.

Auch ist es möglich, das Befestigungsband als geschlossenes Band auszugestalten, welches das Gehäuse des Spenders vollständig umgibt. Das Band kann bei einer solchen Gestaltung insbesondere in einem umfänglich geschlossenen und durchgehenden Schacht des Gehäuses der Auswerteeinheit angeordnet sein und so sicher an der Auswerteeinheit befestigt sein.

Insbesondere bei einem wenig elastischen Band kann es zweckmäßig sein, dass das Befestigungsband zwei Bandabschnitte umfasst, die jeweils an einer Haupteinheit oder einem Gehäuse der Auswerteeinheit befestigt sind. Die beiden Bandabschnitte sind im Bereich eines Verschlusses wie beispielsweise eines Magnet- oder Klettverschlusses miteinander verbindbar, um die Auswerteinheit hierdurch am Gehäuse des Spenders zu befestigen. Das Befestigungsband kann auch als längenveränderliches Band ausgebildet sein, welches mittels eines Verschlusses in einer gewünschten Längenkonfiguration festgestellt werden kann. Es kann zweckmäßig sein, wenn der Verschluss zusammenwirkende Verschlusselemente an beiden Bandabschnitten aufweist, wobei an einem der Bandabschnitte vorzugsweise eine Mehrzahl von Verschlusselementen vorgesehen sind, so dass je nach Umfang des Gehäuses jeweils ein passendes Verschlusselement zum Zusammenfügen der Bandabschnitte gewählt werden kann.

Zusätzlich zum Band kann auch an der Außenseite des Gehäuses des Spenders, insbesondere an dessen Speicherkörper, eine magnetische oder magnetisierbare Haltefläche vorgesehen sein. Dies gestattet es, im Bereich dieser Haltefläche die Auswerteeinheit anzulegen, wobei die Anlageseite der Auswerteeinheit hierfür korrespondierend zur gehäuseseitigen Haltefläche magnetisch oder magnetisierbar ausgebildet ist.

Vorzugsweise ist am Gehäuse eine magnetisierbare und insbesondere eine metallische Haltefläche vorgesehen, während an der Auswerteeinheit mindestens ein Permanentmagnet vorgesehen ist, aber auch die umgekehrte Konstellation ist möglich. Auch ist eine Gestaltung möglich, bei der sowohl die Haltefläche als auch magnetische Gestaltung der Auswerteeinheit mit Permanentmagneten realisiert ist.

Als Permanentmagneten kommen insbesondere Neodym-Magneten in Frage, da solche Magneten eine starke Magnetkraft bereitstellen und daher für die sichere Befestigung der Auswerteeinheit am Gehäuse des Spenders besonders von Vorteil sind.

Die am Gehäuse vorgesehene Haltefläche kann auf verschiedene Arten bereitgestellt werden. Insbesondere bevorzugt ist es, wenn die Haltefläche im Bereich eines beschrifteten Etiketts vorgesehen ist. Sie kann unmittelbar das Etikett bilden oder mittels des Etiketts an einer Gehäusefläche des Gehäuses festgeklebt sein. Die Haltefläche kann auch unabhängig vom Etikett selbstständig am Gehäuse mittels einer Klebeverbindung angebracht sein. Auch sind Gestaltungen möglich, bei denen die Haltefläche durch eine Haltehülse oder Halteklemme gebildet wird, die klemmend am Gehäuse des Spenders angebracht ist.

Eine weitere, nicht beanspruchte Möglichkeit der Befestigung der Auswerteeinheit am Gehäuse sieht vor, dass die Auswerteeinheit mindestens zwei und vorzugsweise mindestens drei elastisch auslenkbare Haltearme aufweist, die das Gehäuse, insbesondere einen Speicherkörper des Gehäuses, umgreifen und damit einen klemmenden Halt der Auswerteinheit am Gehäuse bewirken. Vorzugsweise sind mindestens drei Haltearme vorgesehen, von denen zwei Haltearme sich in einer ersten Erstreckungsrichtung erstrecken und von denen der dritte Haltearm zwischen diesen beiden Haltearmen angeordnet ist und sich in entgegengesetzter Erstreckungsrichtung erstreckt.

Die Auswerteeinheit kann ein Gehäuse aufweisen, welches nicht-modular aufgebaut ist und für den Anwender nicht weiter zerlegbar. Eine besondere Ausgestaltung eines erfindungsgemäßen Spenders und einer erfindungsgemäßen Auswerteeinheit sieht jedoch vor, dass die Auswerteeinheit eine Befestigungseinheit und eine Elektronikeinheit umfasst, die getrennte Baueinheiten darstellen. Die Befestigungseinheit ist dabei zur lösbaren Anbringung am Gehäuse ausgebildet und bildet die Anlageseite der Auswerteeinheit und die daran vorgesehenen Anlagerippen. Die hiervon trennbare Elektronikeinheit ist zur lösbaren Anbringung an der Befestigungseinheit vorgesehen. Die Elektronikeinheit umfasst vorzugsweise die Gesamtheit der elektronischen Komponenten der Auswerteeinheit, mindestens jedoch die Batterie, den Prozessor sowie mindestens einen Sensor.

Die Zweiteilung der Auswerteeinheit gestattet es, die Elektronikeinheit mit unterschiedlichen Befestigungseinheiten zu verwenden, von denen nicht alle in oben beschriebener Weise über Anlagerippen verfügen und somit nicht erfindungsgemäß sind.

Es können jedoch auch unterschiedliche Befestigungseinheiten vorgesehen sein, die jeweils über Anlagerippen verfügen, wobei diese unterschiedlich ausgestaltet und insbesondere beabstandet sind, so dass die Befestigungseinheiten jeweils für Spendergehäuse unterschiedlicher Formgebung geeignet sind.

Die gleiche Elektronikeinheit kann demnach durch Konfiguration mit unterschiedlichen Befestigungseinheiten zur Anbringung an vielen unterschiedlichen Spendern vorgesehen sein.

Die Möglichkeit, die Befestigungseinheit und die Elektronikeinheit zu trennen und in anderer Konfiguration wieder zusammenzusetzen, muss nicht zwingend für den Endanwender gegeben sein. Die genannte Modularität kann auch dafür genutzt werden, um einheitliche Elektronikeinheiten bereits im Rahmen der Herstellung und Bereitstellung bedarfsgerecht mit passenden Befestigungseinheiten zu versehen und ggf. fest zu verbinden.

Besonders bevorzugt ist es allerdings, wenn die Befestigungseinheit zur werkzeuglos lösbaren Anbringung an der Elektronikeinheit ausgebildet ist und damit dem Endanwender selbst die Möglichkeit des Wechsels der Befestigungseinheit offensteht. Insbesondere kann zu diesem Zweck vorgesehen sein, dass die Elektronikeinheit magnetisch an der Befestigungseinheit befestigt ist oder die Elektronikeinheit durch einen Rastmechanismus mit einer elastisch auslenkbaren Rastlasche an der Befestigungseinheit befestigt ist.

Die Befestigungseinheit ist vorzugsweise frei von elektronischen Komponenten. Sie kann jedoch auch Träger von Sensoren sein und/oder ein elektronisch auslesbares Identifikationsmerkmal aufweisen, beispielsweise ein Identifikationsmerkmal, das in einem RFID-Chip der Befestigungseinheit abgelegt ist und durch einen Sensor der Elektronikeinheit auslesbar ist. Hierdurch kann protokolliert werden, mit welcher Befestigungseinheit die Elektronikeinheit verbunden ist.

Die erfindungsgemäße Auswerteeinheit kann im Verkauf zusammen mit dem Spender als Set gehandhabt werden. Die Auswerteinheit kann aber auch separat erworben werden, um sie mit bestehenden Spendern zu verwenden. Von Vorteil kann es daher sein, wenn die elektronische Auswerteeinheit eine Mehrzahl von Befestigungsbändern umfasst, die wahlfrei an der Auswerteinheit, insbesondere an der Befestigungseinheit, anbringbar sind. Auch ist es möglich, dass die Auswerteeinheit über eine Mehrzahl von Befestigungseinheiten verfügt, die wahlfrei an der Elektronikeinheit angebracht werden können, um die Auswerteeinheit zur Anbringung an einem bestimmten Spendertyp zu konfigurieren.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen, die nachfolgend anhand der Figuren erläutert sind.
Fig. 1 bis 3 zeigen exemplarisch einen erfindungsgemäßen Flüssigkeitsspender mit einer erfindungsgemäßen Auswerteeinheit.
Fig. 4 bis 6 verdeutlichen eine erste Konfiguration von Anlagerippen an einer Anlageseite der Auswerteeinheit.
Fig. 7 bis 8 verdeutlichen eine zweite Konfiguration von Anlagerippen an einer Anlageseite der Auswerteeinheit.
Fig. 10 bis 14 zeigen verschiedene Ausgestaltungen von Befestigungsbändern zur Anbringung der Auswerteeinheit am Gehäuse des Spenders.
Fig. 15 bis 17 zeigen die magnetische Anbringung der Auswerteeinheit am Gehäuse des Spenders.
Fig. 18 bis 21 zeigen eine mögliche modulare Ausgestaltung der Auswerteeinheit mit einer Befestigungseinheitund einer Elektronikeinheit sowie Kopplungsmittel zur Kopplung der Elektronikeinheit an die Befestigungseinheit.

### DETAILLIERTE BESCHREIBUNG DER AUSFÜHRUNGSBEISPIELE

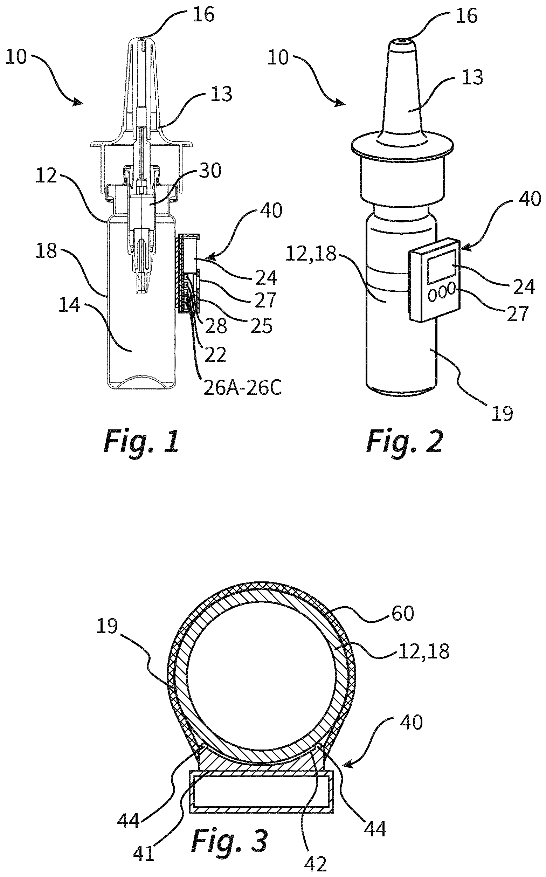

Fig. 1 bis 3 zeigen einen erfindungsgemäßen Spender 10, der zum Austrag pharmazeutischer Flüssigkeiten ausgebildet ist. Der Flüssigkeitsspender 10 ist exemplarisch als Nasalspender ausgebildet und verfügt über ein Applikatorteil mit einem länglichen Nasalapplikator 13, an dessen distalem Ende eine Austragöffnung 16 vorgesehen ist. Der konkrete Spendertyp ist jedoch nur als Beispiel zu verstehen. Die Erfindung umfasst ebenso auch andere Spender für pharmazeutische oder kosmetische Medien und insbesondere Flüssigkeiten, insbesondere pharmazeutische Spender, die zur oralen oder topischen Abgabe von Medikamenten ausgebildet sind.

Durch Niederdrücken des Applikatorteils gegenüber einem Flüssigkeitsspeicher 14 bzw. dem den Flüssigkeitsspeicher bildenden Speicherkörper 18 kann eine Pumpeinrichtung 30 betätigt werden, die Flüssigkeit aus dem Flüssigkeitsspeicher 14 zur Austragöffnung 16 fördert.

Bei einem erfindungsgemäßen Spender 10 ist vorgesehen, dass die Nutzung des Spenders 10 durch den Anwender elektronisch erfasst wird. Hierfür ist eine elektronische Auswerteeinheit 40 vorgesehen. Diese umfasst im vorliegenden Beispiel eine Batterie 25, eine Anzeigeeinrichtung 24, eine Platine mit einem Prozessor 28, Bedienelemente 27 sowie verschiedene Sensoren 26A bis 26C.

Die Sensoren 26A-26C können insbesondere einen Bewegungssensor umfassen, der die Ausrichtung des Spenders und/oder Beschleunigungen erfasst. Weiterhin können die Sensoren 26A-26C ein Mikrofon umfassen, welches Geräusche erfasst, die bei der Handhabung des Spenders 10 auftreten. Die Auswertung der erfassten Daten erfolgt entweder direkt mittels des Prozessors 28 oder ganz oder teilweise auf einem externen System, welches über eine Funkschnittstelle mit der Auswerteeinheit 40 verbunden ist.

Exemplarisch kann der Prozessor 28 dafür ausgebildet sein, im Falle von mittels des Bewegungssensors erfassten Bewegungen des Spenders 10 das Mikrofon zu aktivieren. Mittels des Mikrofons können Geräusche erfasst werden, insbesondere durch das Spendergehäuse 12 übertragene Geräusche. Insbesondere gehen die verschiedenen Phasen eines Pumpvorgangs, also das Ausbringen von Flüssigkeit aus einer Pumpkammer und das erneute Ansaugen von Flüssigkeit aus dem Medienspeicher 14 in die Pumpkammer mit charakteristischen Geräuschen einher, die gut voneinander unterscheidbar sind.

Die Auswerteeinheit 40 ist am Speicherkörper 18 des Spendergehäuses 12 angebracht und zwar an der Mantelfläche 19 des im Wesentlichen zylindrischen Speicherkörpers 18, vorliegend eines kreiszylindrischen Speicherkörpers.

Die Auswerteeinheit 40 umgibt den Speicherkörper 18 nicht, sondern ist rucksackähnlich an einer Seite vorgesehen, so dass sie dem Spender eine asymmetrische Gesamtform verleiht. Die Auswerteeinheit bzw. das den Hauptbestandteil bildende Elektronikgehäuse41 überspannen nureinen vergleichsweise geringen Teil der Mantelfläche, in Umfangsrichtung vorliegend in etwa 90° oder ein Viertel des Umfangs.

Die Auswerteeinheit 40 verfügt über ein Befestigungsband 60, welches den Speicherkörper 18 umgibt und mittels dessen die Auswerteeinheit 40 an den Speicherkörper angepresst wird. Dieses Befestigungsband ist mit beiden Enden am Elektronikgehäuse 41 der Auswerteeinheit angebracht.

Dabei liegt die Auswerteeinheit 40, genauer: deren Elektronikgehäuse 41, nicht vollflächig am Speicherkörper 18 an, sondern im Bereich von Anlagerippen 44, die an der Anlageseite 42 der Auswerteeinheit 40 auf der Rückseite des Elektronikgehäuses 41 vorgesehen sind. In Fig. 3 ist dies anhand einer Schnittdarstellung exemplarisch verdeutlicht.

Durch die Anlagerippen 44 ist der direkte Anlagekontakt zwischen dem Spendergehäuse 12 und der Auswerteeinheit 44 auf die Anlageflächen der Anlagerippen 44 beschränkt, so dass hier eine vergleichsweise große Flächenpressung und ein sicherer Halt erzielt wird. Insbesondere die Auswertungen von Schallwellen, die durch die Nutzung des Spenders bedingt sind und bestimmungsgemäß durch das Mikrofon erfasst werden, ist durch die Anlagerippen 44 in besserer Weise möglich.

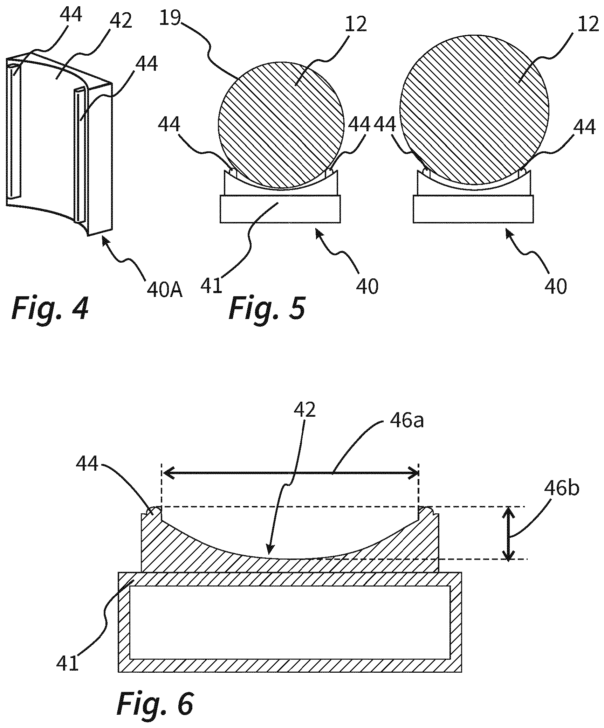

Die Fig. 4 bis 6 zeigen eine erste Konfiguration von Anlagerippen 44. In Fig. 4 ist die Rückseite der Auswerteeinheit 40 dargestellt. Diese Rückseite bildet eine Anlageseite 42, wobei zwei parallele Anlagerippen 44 zueinander ortsfest an der Anlageseite 42 vorgesehen sind. Zwischen den beiden Anlagerippen 44 ist ein zurückgesetzter Zwischenbereich vorgesehen. Wie sich anhand der Fig. 5 ersehen lässt, ermöglichen es die Anlagerippen 44, an Spendergehäusen 12 unterschiedlicher Krümmungsradien anzuliegen. Die gleiche Auswerteeinheit 40 bzw. die gleiche Befestigungseinheit 40A der Auswerteeinheit 40 kann also für unterschiedliche Spender 10 gleichermaßen gut verwendet werden.

Fig. 6 verdeutlicht nochmals die Geometrie der Anlageseite 42. Es ist zu ersehen, dass diese eine grundsätzlich gewölbte konkave Form aufweist und dass die beiden Anlagerippen 44 sich über diese gewölbte konkave Form erheben. Der Abstand 46A entspricht der Breite des Zwischenbereichs. Der Abstand 46B zeigt an, wieweit der Zwischenbereich maximal gegenüber den Anlagerippen 44 zurückgesetzt ist.

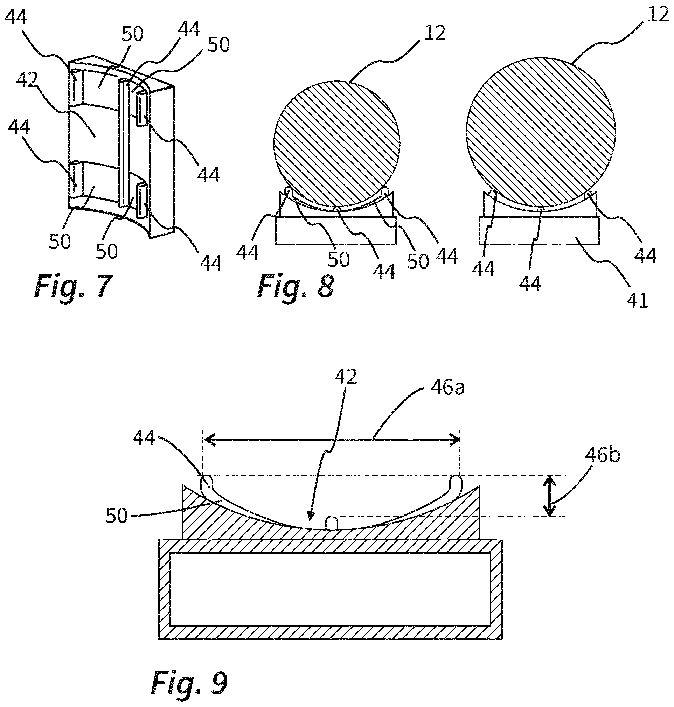

Die Fig. 7 bis 9 zeigen eine zweite Konfiguration von Anlagerippen 44. Wie anhand der Fig. 7 zu ersehen ist, ist die Anlageseite 42 hier mit insgesamt drei Anlagerippen 44 versehen. Eine zentrische Anlagerippe 44 ist fest mit dem Gehäuse der Auswerteeinheit 40 verbunden und gegenüber diesem nicht in relevantem Maße auslenkbar. Zwei seitliche Anlagerippen 44 sind dagegen aus Auslegern 50 vorgesehen, wobei beide seitliche Anlagerippen 44 jeweils aus zwei miteinander fluchtenden Rippensegmenten bestehen, die ihrerseits jeweils an einem Ausleger 50 vorgesehen sind. Die beiden äußeren Anlagerippen können daher federnd ausgelenkt werden, wobei sie sich gegenüber dem Elektronikgehäuse 41 bewegen.

Die Ausleger 50 gestatten es, die außenliegenden Anlagerippen 44 gegen die Federkraft der Ausleger 50 relativ zueinander zu verlagern. Hierdurch kann trotz insgesamt dreier Anlagerippen erreicht werden, dass alle Anlagerippen bei Verwendung der Auswerteeinheit 40 mit unterschiedlichen Spendergehäusen 12 bzw. Speicherkörpern 18 jeweils am Spendergehäuse 12 anliegen. Bei Spendergehäusen 12/Speicherkörpern 18 mit größeren Durchmessern werden die Ausleger 50 elastisch ausgelenkt, wobei dies insbesondere im Kontext einer Befestigung der Auswerteinheit 40 mit einem Befestigungsband 60 von Vorteil sein kann.

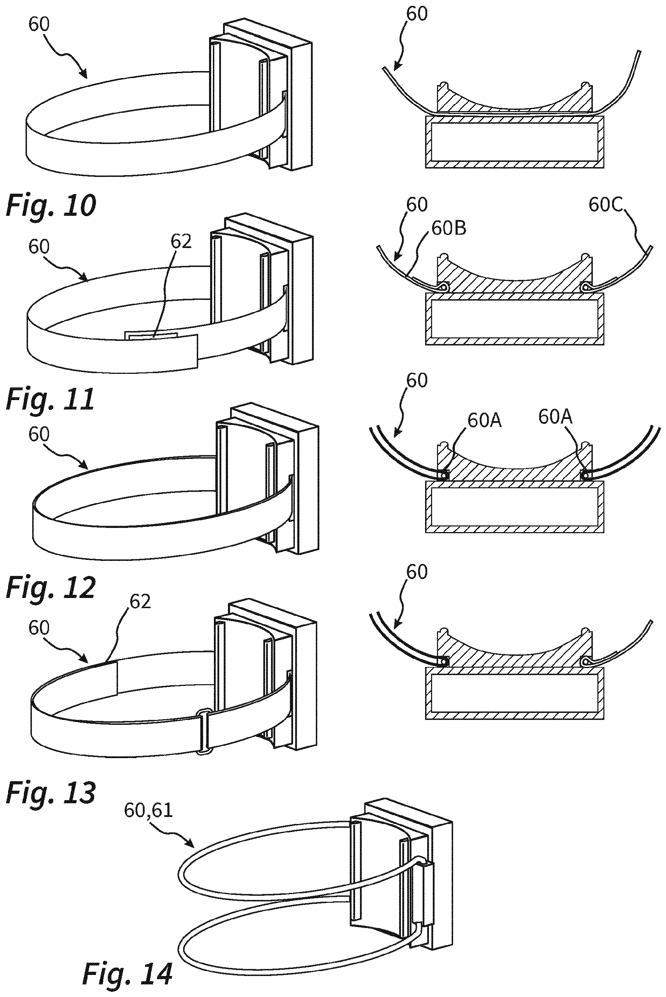

Die Fig. 10 bis 14 zeigen unterschiedliche Konfigurationen eines solchen Befestigungsbandes 60. Die jeweils dargestellten Befestigungsbänder 60 sind an einem Gehäuse der Auswerteeinheit 40 angebracht und werden um das Spendergehäuse 12 herumgelegt.

Bei der Gestaltung der Fig. 10 ist das Befestigungsband 60 als vollständig umlaufendes Befestigungsband 60 gestaltet, welches im Bereich des Gehäuses der Auswerteeinheit 40 durch einen Schacht 64 zwischen einer Elektronikeinheit 40B und der Befestigungseinheit 40A geführt ist. Das Befestigungsband ist elastisch ausgebildet und kann beispielsweise aus Gummi gefertigt sein. Es gestattet die Anbringung an Spendergehäusen unterschiedlichen Umfangs.

Bei der Gestaltung gemäß Fig. 11 weist das Befestigungsband zwei Bandabschnitte 60B, 60C auf, die jeweils am Gehäuse der Auswerteeinheit 40 befestigt sind. Die Bandabschnitte 60B, 60C können von beiden Seiten um das Spendergehäuse 12 herumgelegt werden und mittels eines Verschlusses 62 miteinander verbunden werden. Der Verschluss 62 kann beispielsweise ein Magnetverschluss sein.

Bei der Gestaltung der Fig. 12 findet ein geschlossenes Befestigungsband 60 Verwendung, welches jedoch doppelt um das Spendergehäuse 12 herumgelegt wird und im Bereich des Gehäuses der Auswerteeinheit in Form zweier Schlaufen 60A gelegt ist, die am Gehäuse der Auswerteeinheit 40 befestigt sind.

Die Gestaltung der Fig. 13 sieht vor, dass das dort Verwendung findende Befestigungsband 60 längenveränderlich ausgestaltet ist und in der für das Spendergehäuse richtigen Einstellung mittels eines Verschlusses 62 gesichert werden kann.

Die Gestaltung der Fig. 14 ähnelt jener der Fig. 12. Auch hier findet ein geschlossenes Befestigungsband 60 Verwendung, welches zweisträngig um das Spendergehäuse 12 herumgelegt wird und welches im Bereich seiner Enden zwei Schlaufen ausbildet, die am Gehäuse der Auswerteeinheit 40 befestigt sind. Abweichend von der Gestaltung der Fig. 12, bei der ein flaches Befestigungsband 60 genutzt wird, ist das Befestigungsband 60 der Fig. 14 als O-Ring mit kreisrundem Querschnitt gebildet.

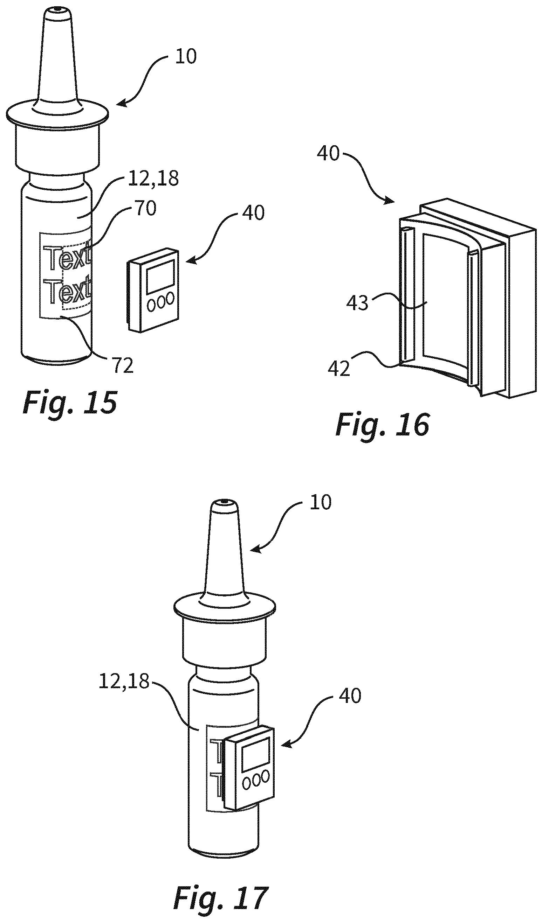

Die Fig. 15 bis 17 zeigen eine alternative, nicht erfindungsgemäße Form der Befestigung der Auswerteeinheit 40 am Spendergehäuse 12. Hier ist vorgesehen, dass am Speicherkörper 18 des Spenders 10 ein Etikett 72 angebracht ist. In dieses Etikett 72 ist eine magnetisierbare Haltefläche 70 integriert, die beispielsweise aus einem magnetisierbaren Metall besteht.

Korrespondierend hierzu ist an der Anlageseite 42 der Auswerteeinheit 40 ein Permanentmagnet 43 vorgesehen. Wird die Auswerteeinheit 40 in den Bereich der Haltefläche 70 geführt, so wird sie magnetisch angezogen und verbindet sich somit magnetisch mit dem Speicherkörper 18, wie in Fig. 17 dargestellt ist. Der Permanentmagnet 43 ist vorzugsweise als Neodym-Magnet ausgebildet, um eine für einen sicheren Halt ausreichende Haltekraft zu verursachen.

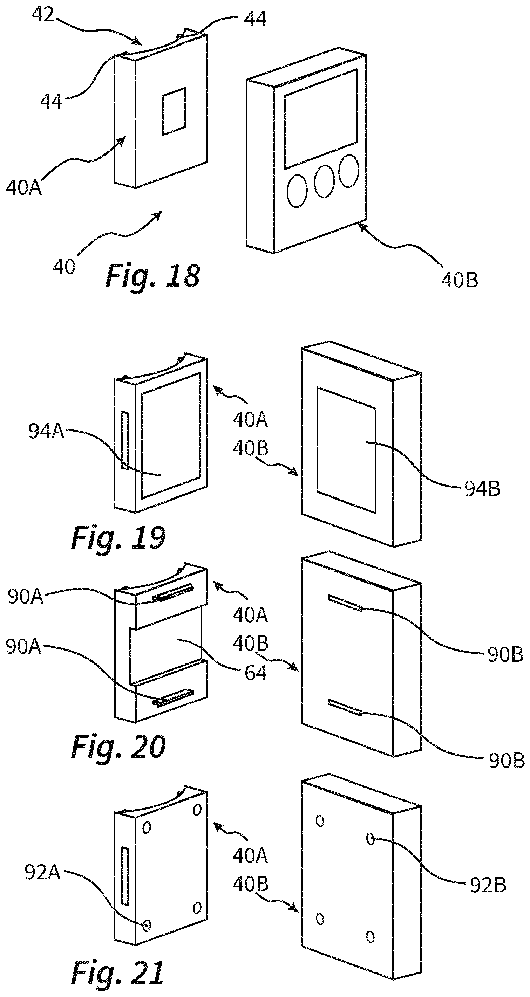

Die Fig. 18 bis 21 verdeutlichen die Möglichkeit, die Auswerteeinheit 40 modular zu gestalten. Wie anhand der Fig. 18 verdeutlicht, untergliedert sich die Auswerteeinheit 40 in solchen Fällen in eine Befestigungseinheit 40A, an der die Anlageseite 42 sowie die Anlagerippen 44 vorgesehen sind, sowie in eine Elektronikeinheit 40B, die die überwiegende Zahl an elektrischen/elektronischen Komponenten aufweist, vorzugsweise die Gesamtheit der elektronischen Komponenten. Es kann allerdings vorgesehen sein, dass die Befestigungseinheit 40A über ein elektronischen Identifikationsmittel wie einen RFID-Chip verfügt, damit die Elektronikeinheit 40B erkennen kann, ob sie mit einer Befestigungseinheit 40A verbunden ist bzw. mit welcher Befestigungseinheit 40A sie verbunden ist.

Die Teileinheiten 40A, 40B sind vorzugsweise werkzeuglos miteinander koppelbar. Bei der Gestaltung der Fig. 19 sind die beiden Teileinheiten mit magnetischen oder magnetisierbaren Kopplungsflächen 94A, 94B verbunden.

Bei der Gestaltung der Fig. 20 sind Rastlaschen 90A an der Befestigungseinheit 40A vorgesehen, die in Rastöffnungen 90B an der Elektronikeinheit 40B verrasten. Vorzugsweise sind die Rastlaschen 90A abweichend von der Gestaltung der Fig. 20 manuell auslenkbar, um die Verbindung wieder trennen zu können. Es kann jedoch auch vorgesehen sein, dass die Verbindung der Elektronikeinheit 40B mit der Befestigungseinheit 40A bestimmungsgemäß unlösbar ist, so dass eine entsprechende Kopplung nur einmalig möglich ist, insbesondere bei einer bereits im Zuge der Herstellung stattfindenden Festlegung auf eine bestimmte Befestigungseinheit 40A. Bei der Gestaltung der Fig. 20 ist darüber hinaus ersichtlich, dass an einer der Einheiten, vorliegend an der Befestigungseinheit 40A, eine Vertiefung vorgesehen ist, die der Bildung des Schachtes 64 und der Aufnahme eines Befestigungsbandes 60 dient.

Bei der Gestaltung der Fig. 21 sind Schraublöcher 92A, 92B an der Elektronikeinheit 40B und der Befestigungseinheit 40A vorgesehen, durch die hindurch die beiden Teileinheiten miteinander verschraubt werden können. Diese Art der Verbindung ist üblicherweise nicht dafür vorgesehen, vom Endanwender gelöst zu werden, sondern dient dem Zweck, im Zuge der Herstellung eine einheitliche Elektronikeinheit 40B dauerhaft mit einer Befestigungseinheit 40A verbinden zu können.

## Patentansprüche

1. Spender (10) zum Austrag pharmazeutischer oder kosmetischer Medien mit den folgenden Merkmalen:
a. der Spender (10) weist ein Gehäuse (12) auf, und
b. der Spender (10) weist einen Medienspeicher (14) auf, und
c. der Spender (10) weist eine Austragöffnung (16) auf, durch die Medium aus dem Medienspeicher (14) in eine umgebende Atmosphäre abgegeben werden kann, und
d. der Spender (10) weist eine elektronische Auswerteeinheit (40) zur Erfassung der Nutzung des Spenders auf, und
e. die elektronische Auswerteeinheit (40) ist zur lösbaren Anbringung am Gehäuse (12) des Spenders (10) ausgebildet, und
f. die elektronische Auswerteeinheit (40) weist eine Anlageseite (42) auf, die im angebrachten Zustand der Auswerteeinheit (40) in Richtung des Gehäuses (12) weist, und
g. die elektronische Auswerteeinheit (40) weist an der Anlageseite (42) zwei zueinander parallele Anlagerippen (44) zur Anlage am Gehäuse (12) auf, und zwar zur Anlage an einem Speicherkörper (18) des Medienspeichers (14), wobei die Anlagerippen (44) einen Abstand (46A) voneinander aufweisen, der nicht mehr als 80% des Durchmessers des Speicherkörpers (19) beträgt,
**gekennzeichnet durch** das folgende Merkmal:
h. die elektronische Auswerteeinheit (40) weist ein formflexibles Befestigungsband (60) auf, welches an der Auswerteeinheit (40) angebracht ist und das Gehäuse (12), und zwar den Speicherkörper (18), umgibt.

2. Spender (10) nach Anspruch 1 mit den folgenden weiteren Merkmalen:
a. die elektronische Auswerteeinheit (40) ist zur lösbaren Anbringung an einer Mantelfläche (19) des Speicherkörper (18) des Gehäuses vorgesehen, wobei die Mantelfläche (19) vorzugsweise eine kreiszylindrische Mantelfläche (19) ist, und
b. die elektronische Auswerteinheit (40) weist ein Elektronikgehäuse (41) auf, innerhalb dessen die elektronischen Komponenten der Auswerteinheit (40) angeordnet sind, an welchem die Anlageseite (42) und die Anlagerippen (44) als vertikal erstreckte Anlagerippen (44) vorgesehen sind, und
c. das Elektronikgehäuse (40) überdeckt in Umfangsrichtung höchstens 50% der Mantelfläche (19) des Speicherkörpers (18), insbesondere vorzugsweise höchstens 40% der Mantelfläche (19) des Speicherkörpers (18).

3. Spender (10) nach Anspruch 2 mit dem folgenden weiteren Merkmal:
a. die Anlagerippen (44) verbleiben bei Betätigung des Spenders (10) ortsfest zur Mantelfläche (19) des Speicherkörpers (18).

4. Spender (10) nach einem der vorstehenden Ansprüche mit den folgenden weiteren Merkmalen:
a. die elektronische Auswerteeinheit (40) ist zur lösbaren Anbringung an einer Mantelfläche (19) des Speicherkörper (18) des Gehäuses vorgesehen, wobei die Mantelfläche (19) vorzugsweise eine kreiszylindrische Mantelfläche (19) ist, und
b. die Anlagerippen (44) befinden sich bezogen auf die Umfangsrichtung des Speicherkörpers (18) in einem Teilbereich der Mantelfläche (19) von maximal 40% des Umfangs des Speicherkörpers (18), vorzugsweise in einem Teilbereich der Mantelfläche von maximal 25% des Umfangs des Speicherkörpers (18).

5. Spender (10) nach einem der vorstehenden Ansprüche mit mindestens einem der folgenden weiteren Merkmale:
a. die Anlagerippen (44) weisen einen Abstand (46A) voneinander von mindestens 8 mm auf, und/oder
b. die Anlagerippen (44) weisen einen Abstand (46A) voneinander von maximal 20 mm auf, und/oder
c. die Anlagerippen (44) erheben sich gegenüber einem zwischen ihnen liegenden Zwischenbereich (48) um einen Abstand (46B) von mindestens 1,5 mm.

6. Spender (10) nach einem der vorstehenden Ansprüche mit dem folgenden weiteren Merkmal:
a. mindestens eine Anlagerippe (44) ist an einem elastischen Ausleger (50) angebracht.

7. Spender nach einem der vorstehenden Ansprüche mit dem folgenden weiteren Merkmal:
a. die Anlagerippen (44) werden durch mehrere zueinander fluchtende Rippensegmente gebildet, die vorzugsweise jeweils an einem elastischen Ausleger (50) vorgesehen sind.

8. Spender (10) nach einem der vorstehenden Ansprüche mit dem folgenden weiteren Merkmal:
a. die elektronische Auswerteinheit (40) weist ein Elektronikgehäuse (41) auf, und
b. das formflexible Befestigungsband (60) ist als separates Element und vorzugsweise aus einem anderen Material als das Elektronikgehäuse (41) ausgebildet.

9. Spender (10) nach Anspruch 8 mit dem folgenden weiteren Merkmal:
a. das Befestigungsband (60) ist als elastisches Band ausgebildet,
insbesondere vorzugsweise mit mindestens einem der folgenden zusätzlichen Merkmale:
b. das Befestigungsband (60) ist als O-Ring (61) ausgebildet, und/oder
c. das Befestigungsband (60) ist als geschlossenes Band ausgebildet, welches in Form eines Doppelbandes das Gehäuse umgibt und im Bereich zweier umlenkender Schlaufen (60A) an einer Haupteinheit der Auswerteeinheit befestigt ist, oder
d. das Befestigungsband (60) ist als geschlossenes Band ausgebildet, welches das Gehäuse vollständig umgebend an diesem angebracht ist.

10. Spender (10) nach einem der vorstehenden Ansprüche mit den folgenden weiteren Merkmalen:
a. das Befestigungsband (60) umfasst zwei Bandabschnitte (60B, 60C), die jeweils an einer Haupteinheit der Auswerteeinheit (40) befestigt sind und die miteinander im Bereich eines Verschlusses (62) verbunden sind, oder
b. das Befestigungsband (60) ist als längenveränderliches Band ausgebildet, welches mittels eines Verschlusses (62) in einer gewünschten Längenkonfiguration festgestellt werden kann,
vorzugsweise mit einem der folgenden zusätzlichen Merkmale:
c. der Verschluss (62) ist als Klettverschluss ausgebildet, und/oder
d. der Verschluss weist zusammenwirkende Verschlusselemente an beiden Bandabschnitten auf, wobei an einem der Bandabschnitte vorzugsweise eine Mehrzahl von Verschlusselementen vorgesehen sind, und/oder
e. der Verschluss (62) ist als magnetischer Verschluss ausgebildet.

11. Spender (10) nach einem der vorstehenden Ansprüche mit den folgenden weiteren Merkmalen:
a. die Auswerteeinheit (40) umfasst eine Befestigungseinheit (40A) und eine Elektronikeinheit (40B), und
b. die Befestigungseinheit (40A) ist zur lösbaren Anbringung am Gehäuse (12) ausgebildet, und
c. die Elektronikeinheit (40B) ist zur lösbaren Anbringung an der Befestigungseinheit (40A) vorgesehen.

12. Spender (10) nach Anspruch 11 mit mindestens einem der folgenden zusätzlichen Merkmale:
a. die Befestigungseinheit (40A) ist zur werkzeuglos lösbaren Anbringung am Gehäuse (12) ausgebildet, und/oder
b. die Elektronikeinheit (40B) ist magnetisch an der Befestigungseinheit (40A) befestigt, und/oder
c. die Elektronikeinheit (40B) ist durch einen Rastmechanismus mit einer elastisch auslenkbaren Rastlasche (90) an der Befestigungseinheit (40A) befestigt, und/oder
d. die Befestigungseinheit (40A) weist ein Identifikationsmerkmal auf, insbesondere abgelegt in einem RFID-Chip der Befestigungseinheit, welcher durch einen Sensor (26B) der Elektronikeinheit (40B) auslesbar ist.

13. Spender (10) nach einem der vorstehenden Ansprüche mit mindestens einem der folgenden weiteren Merkmale:
a. die Auswerteeinheit (40) umfasst mindestens einen Sensor (26A, 26B, 26C), insbesondere einen Bewegungssensor (26A) und/oder ein Mikrofon (26C), und/oder
b. die Auswerteeinheit (40) umfasst eine Ausgabeeinrichtung (24), insbesondere in Form mindestens einer LED, einer Anzeigeeinrichtung (24), eines Vibrators und/oder eines Lautsprechers,
c. die Auswerteeinheit umfasst ein Funkmodul (22), insbesondere ein Bluetooth- und/oder WIFI-Funkmodul (22), und/oder
d. die Auswerteeinheit umfasst eine Energiequelle (25), insbesondere in Form einer Batterie.

14. Spender (10) nach einem der vorstehenden Ansprüche mit dem folgenden weiteren Merkmal:
a. der Spender (10) ist als Flüssigkeitsspender (10) ausgebildet, wobei der Medienspeicher (14) als Flüssigkeitsspeicher (14) ausgebildet ist,
insbesondere mit mindestens einem der folgenden zusätzlichen Merkmale:
b. der Spender ist als Quetschflaschenspender ausgebildet und weist einen elastisch verformbaren Speicherkörper des Flüssigkeitsspeichers auf, durch dessen manuelle Komprimierung Flüssigkeit ausgetragen werden kann, oder
c. der Spender (10) ist als Pumpspender ausgebildet, der über eine manuell betätigbare Pumpeinrichtung (30) verfügt, die Flüssigkeit aus dem Flüssigkeitsspeicher (14) zur Austragöffnung (16) fördert, und/oder
d. der Spender (10) ist als Sprühspender ausgebildet, und/oder
e. der Spender ist als Inhalator ausgebildet, insbesondere als Spender mit einem zur Abgabe einer Dosis der Flüssigkeit niederdrückbarem Container,
f. der Spender ist als Tropfenspender ausgebildet, insbesondere mit einer die Austragöffnung umgebenden Tropfenbildungsfläche, und/oder
g. der Spender ist als Nasalspender ausgebildet, insbesondere mit einem langgestreckten Nasalapplikator, an dessen distalem Ende die Austragöffnung vorgesehen ist, und/oder
h. der Medienspeicher (14) des Spenders weist ein Volumen von maximal 200 ml auf, vorzugsweise von maximal 100 ml, und/oder
i. der Medienspeicher (14) des Spenders ist mit einer pharmazeutischen Flüssigkeit befüllt.

15. Auswerteeinheit für einen Spender nach einem der vorstehenden Ansprüche mit den folgenden Merkmalen:
a. die Auswerteeinheit (40) ist zur lösbaren Anbringung an einem Spender (10) ausgebildet, und
b. die elektronische Auswerteeinheit (40) ist zur Erfassung der Nutzung des Spenders ausgebildet und weist hierfür mindestens einen Sensor (26A, 26C) auf, und
c. die elektronische Auswerteeinheit (40) weist eine Anlageseite (42) auf, die im angebrachten Zustand der Auswerteeinheit (40) in Richtung eines Gehäuses (12) des Spenders (10) weist, und
d. die elektronische Auswerteeinheit (40) weist an der Anlageseite (42) zwei zueinander parallele Anlagerippen (44) zur Anlage am Gehäuse (12) auf, und zwar zur Anlage an einem Speicherkörper (18) eines Medienspeichers (14) des Spenders (10), wobei die Anlagerippen (44) einen Abstand (46A) voneinander aufweisen, der nicht mehr als 80% des Durchmessers des Speicherkörpers (19) beträgt,
**gekennzeichnet durch** das folgende Merkmal:
e. die elektronische Auswerteeinheit (40) weist ein formflexibles Befestigungsband (60) auf, welches bei Benutzung an der Auswerteeinheit (40) angebracht ist und das Gehäuse (12), und zwar den Speicherkörper (18), umgibt, vorzugsweise mit mindestens einem der folgenden zusätzlichen Merkmale:
f. die elektronische Auswerteeinheit (40) umfasst eine Mehrzahl von Befestigungsbändern, die wahlfrei an der Auswerteinheit (40), insbesondere an der Befestigungseinheit (40A) anbringbar sind, und/oder
g. die Auswerteeinheit (40) umfasst eine Mehrzahl von Befestigungseinheiten (40A), die wahlfrei an der Elektronikeinheit (40B) angebracht werden können.

## Claims

1. Dispenser (10) for discharging pharmaceutical or cosmetic media, having the following features:
a. the dispenser (10) has a housing (12), and
b. the dispenser (10) has a media reservoir (14), and
c. the dispenser (10) has a discharge opening (16) through which medium from the media reservoir (14) can be delivered into a surrounding atmosphere, and
d. the dispenser (10) has an electronic evaluation unit (40) for detecting the use of the dispenser, and
e. the electronic evaluation unit (40) is designed for detachable attachment to the housing (12) of the dispenser (10), and
f. the electronic evaluation unit (40) has a bearing side (42) which in the attached state of the evaluation unit (40) faces in the direction of the housing (12), and
g. the electronic evaluation unit (40) has on the bearing side (42) two mutually parallel bearing ribs (44) for bearing against the housing (12), specifically for bearing against a reservoir body (18) of the media reservoir (14), with the bearing ribs (44) being at a distance (46A) from one another that is not more than 80% of the diameter of the reservoir body (18),
**characterized by** the following feature:
h. the electronic evaluation unit (40) has a dimensionally flexible fastening strap (60) which is attached to the evaluation unit (40) and surrounds the housing (12), specifically the reservoir body (18).

2. Dispenser (10) according to Claim 1, having the following further features:
a. the electronic evaluation unit (40) is intended for detachable attachment to a lateral surface (19) of the reservoir body (18) of the housing, with the lateral surface (19) preferably being a circular-cylindrical lateral surface (19), and
b. the electronic evaluation unit (40) has an electronics housing (41), within which the electronic components of the evaluation unit (40) are arranged and on which the bearing side (42) and the bearing ribs (44) are provided as vertically extending bearing ribs (44), and
c. the electronics housing (40) covers in the circumferential direction at most 50% of the lateral surface (19) of the reservoir body (18), in particular preferably at most 40% of the lateral surface (19) of the reservoir body (18).

3. Dispenser (10) according to Claim 2, having the following further feature:
a. the bearing ribs (44) remain fixed in place in relation to the lateral surface (19) of the reservoir body (18) when the dispenser (10) is actuated.

4. Dispenser (10) according to one of the preceding claims, having the following further features:
a. the electronic evaluation unit (40) is intended for detachable attachment to a lateral surface (19) of the reservoir body (18) of the housing, with the lateral surface (19) preferably being a circular-cylindrical lateral surface (19), and
b. the bearing ribs (44) are situated, in relation to the circumferential direction of the reservoir body (18), in a partial region of the lateral surface (19) of at most 40% of the circumference of the reservoir body (18), preferably in a partial region of the lateral surface of at most 25% of the circumference of the reservoir body (18).

5. Dispenser (10) according to one of the preceding claims, having at least one of the following further features:
a. the bearing ribs (44) are at a distance (46A) from one another of at least 8 mm, and/or
b. the bearing ribs (44) are at a distance (46A) from one another of at most 20 mm, and/or
c. the bearing ribs (44) rise up by a distance (46B) of at least 1.5 mm with respect to an intermediate region (48) lying between them.

6. Dispenser (10) according to one of the preceding claims, having the following further feature:
a. at least one bearing rib (44) is attached to an elastic extension arm (50).

7. Dispenser according to one of the preceding claims, having the following further feature:
a. the bearing ribs (44) are formed by a number of mutually aligned rib segments, which are preferably in each case provided on an elastic extension arm (50).

8. Dispenser (10) according to one of the preceding claims, having the following further feature:
a. the electronic evaluation unit (40) has an electronics housing (41), and
b. the dimensionally flexible fastening strap (60) is designed as a separate element and preferably from a material other than that of the electronics housing (41).

9. Dispenser (10) according to Claim 8, having the following further feature:
a. the fastening strap (60) is designed as an elastic strap,
in particular preferably having at least one of the following additional features:
b. the fastening strap (60) is designed as an O-ring (61), and/or
c. the fastening strap (60) is designed as a closed strap, which in the form of a double strap surrounds the housing and is fastened in the region of two deflecting loops (60A) to a main unit of the evaluation unit, or
d. the fastening strap (60) is designed as a closed strap, which is attached to the housing so as to completely surround it.

10. Dispenser (10) according to one of the preceding claims, having the following further features:
a. the fastening strap (60) comprises two strap portions (60B, 60C), which are in each case fastened to a main unit of the evaluation unit (40) and which are connected to each other in the region of a closure (62), or
b. the fastening strap (60) is designed as a variable-length strap which can be established in a desired length configuration by means of a closure (62),
preferably having one of the following additional features:
c. the closure (62) is designed as a hook-and-loop fastener, and/or
d. the closure has interacting closure elements on both strap portions, with a plurality of closure elements preferably being provided on one of the strap portions, and/or
e. the closure (62) is designed as a magnetic closure.

11. Dispenser (10) according to one of the preceding claims, having the following further features:
a. the evaluation unit (40) comprises a fastening unit (40A) and an electronics unit (40B), and
b. the fastening unit (40A) is designed for detachable attachment to the housing (12), and
c. the electronics unit (40B) is intended for detachable attachment to the fastening unit (40A).

12. Dispenser (10) according to Claim 11, having at least one of the following additional features:
a. the fastening unit (40A) is designed for attachment to the housing (12) such that it can be detached without tools, and/or
b. the electronics unit (40B) is magnetically fastened on the fastening unit (40A), and/or
c. the electronics unit (40B) is fastened on the fastening unit (40A) by means of a latching mechanism having an elastically deflectable latching lug (90), and/or
d. the fastening unit (40A) has an identification feature, in particular stored in an RFID chip of the fastening unit, which can be read out by a sensor (26B) of the electronics unit (40B).

13. Dispenser (10) according to one of the preceding claims, having at least one of the following further features:
a. the evaluation unit (40) comprises at least one sensor (26A, 26B, 26C), in particular a movement sensor (26A) and/or a microphone (26C), and/or
b. the evaluation unit (40) comprises an output device (24), in particular in the form of at least one LED, a display device (24), a vibrator and/or a loudspeaker,
c. the evaluation unit comprises a radio module (22), in particular a Bluetooth and/or WIFI radio module (22), and/or
d. the evaluation unit comprises an energy source (25), in particular in the form of a battery.

14. Dispenser (10) according to one of the preceding claims, having the following further feature:
a. the dispenser (10) is designed as a liquid dispenser (10), with the media reservoir (14) being designed as a liquid reservoir (14),
in particular having at least one of the following additional features:
b. the dispenser is designed as a squeeze-bottle dispenser and has an elastically deformable reservoir body of the liquid reservoir, by the manual compression of which liquid can be discharged, or
c. the dispenser (10) is designed as a pumping dispenser which has a manually actuable pumping device (30) which conveys liquid from the liquid reservoir (14) to the discharge opening (16), and/or
d. the dispenser (10) is designed as a spray dispenser, and/or
e. the dispenser is designed as an inhaler, in particular as a dispenser with a container which can be depressed for delivering a dose of the liquid,
f. the dispenser is designed as a drop dispenser, in particular with a drop-formation surface surrounding the discharge opening, and/or
g. the dispenser is designed as a nasal dispenser, in particular with an elongate nasal applicator, at the distal end of which the discharge opening is provided, and/or
h. the media reservoir (14) of the dispenser has a volume of at most 200 ml, preferably of at most 100 ml, and/or
i. the media reservoir (14) of the dispenser is filled with a pharmaceutical liquid.

15. Evaluation unit for a dispenser according to one of the preceding claims, having the following features:
a. the evaluation unit (40) is designed for detachable attachment to a dispenser (10), and
b. the electronic evaluation unit (40) is designed for detecting the use of the dispenser and for this has at least one sensor (26A, 26C), and
c. the electronic evaluation unit (40) has a bearing side (42) which in the attached state of the evaluation unit (40) faces in the direction of a housing (12) of the dispenser (10), and
d. the electronic evaluation unit (40) has on the bearing side (42) two mutually parallel bearing ribs (44) for bearing against the housing (12), specifically for bearing against a reservoir body (18) of a media reservoir (14) of the dispenser (10), with the bearing ribs (44) being at a distance (46A) from one another that is not more than 80% of the diameter of the reservoir body (18),
**characterized by** the following feature:
e. the electronic evaluation unit (40) has a dimensionally flexible fastening strap (60) which, when in use, is attached to the evaluation unit (40) and surrounds the housing (12), specifically the reservoir body (18), preferably having at least one of the following additional features:
f. the electronic evaluation unit (40) comprises a plurality of fastening straps which can be optionally attached to the evaluation unit (40), in particular to the fastening unit (40A), and/or
g. the evaluation unit (40) comprises a plurality of fastening units (40A) which can be optionally attached to the electronics unit (40B).

## Revendications

1. Distributeur (10) pour l'évacuation de milieux pharmaceutiques ou cosmétiques, présentant les caractéristiques suivantes :
a. le distributeur (10) comporte un boîtier (12), et
b. le distributeur (10) comporte un réservoir de milieu (14), et
c. le distributeur (10) présente une ouverture d'évacuation (16) à travers laquelle le milieu peut être déchargé du réservoir de milieu (14) dans une atmosphère environnante, et
d. le distributeur (10) comporte une unité d'évaluation électronique (40) pour détecter l'utilisation du distributeur, et
e. l'unité d'évaluation électronique (40) est conçue pour être fixée de manière amovible au boîtier (12) du distributeur (10), et
f. l'unité d'évaluation électronique (40) présente une face de butée (42) qui, lorsque l'unité d'évaluation (40) est montée, est orientée en direction du boîtier (12), et
g. l'unité électronique d'évaluation (40) présente, sur la face de butée (42), deux nervures de butée (44) parallèles l'une à l'autre destinées à venir en butée contre le boîtier (12), notamment pour être en butée contre un corps de réservoir (18) du réservoir de milieu (14), les nervures de butée (44) étant à une distance (46A) les unes des autres qui ne dépasse pas 80% du diamètre du corps de réservoir (18),
**caractérisé par** la particularité suivante :
h. l'unité d'évaluation électronique (40) comporte une bande de fixation (60) flexible, qui est fixée à l'unité d'évaluation (40) et entoure le boîtier (12), en particulier le corps de réservoir (18).

2. Distributeur (10) selon la revendication 1, présentant les caractéristiques supplémentaires suivantes :
a. l'unité d'évaluation électronique (40) est prévue pour être fixée de manière amovible à une surface extérieure (19) du corps de réservoir (18) du boîtier, la surface extérieure (19) étant de préférence une surface extérieure cylindrique circulaire (19), et
b. l'unité d'évaluation électronique (40) comporte un boîtier électronique (41) dans lequel sont disposés les composants électroniques de l'unité d'évaluation (40), sur lequel sont prévues la face de butée (42) et les nervures de butée (44) sous la forme de nervures de butée (44) s'étendant verticalement, et
c. le boîtier électronique (40) recouvre au maximum 50 % de la surface extérieure (19) du corps de réservoir (18), en particulier de préférence au maximum 40 % de la surface extérieure (19) du corps de réservoir (18), dans la direction circonférentielle.

3. Distributeur (10) selon la revendication 2, présentant la caractéristique supplémentaire suivante :
a. les nervures de butée (44) restent fixes par rapport à la surface extérieure (19) du corps de réservoir (18) lors de l'actionnement du distributeur (10).

4. Distributeur (10) selon l'une quelconque des revendications précédentes, présentant les caractéristiques supplémentaires suivantes :
a. l'unité d'évaluation électronique (40) est prévue pour être fixée de manière amovible à une surface extérieure (19) du corps de réservoir (18) du boîtier, la surface extérieure (19) étant de préférence une surface extérieure cylindrique circulaire (19), et
b. les nervures de butée (44) sont situées, par rapport à la direction circonférentielle du corps de réservoir (18), dans une zone partielle de la surface extérieure (19) d'au plus 40 % de la circonférence du corps de réservoir (18), de préférence dans une zone partielle de la surface extérieure d'au plus 25 % de la circonférence du corps de réservoir (18).

5. Distributeur (10) selon l'une quelconque des revendications précédentes, présentant au moins l'une des autres caractéristiques suivantes :
a. les nervures de butée (44) sont espacées les unes des autres d'une distance (46A) d'au moins 8 mm, et/ou
b. les nervures de butée (44) sont espacées les unes des autres d'une distance (46A) inférieure ou égale à 20 mm, et/ou
c. les nervures de butée (44) s'élèvent par rapport à une zone intermédiaire (48) située entre elles d'une distance (46B) d'au moins 1,5 mm.

6. Distributeur (10) selon l'une quelconque des revendications précédentes, présentant la caractéristique supplémentaire suivante :
a. au moins une nervure de butée (44) est fixée à une flèche élastique (50).

7. Distributeur (10) selon l'une quelconque des revendications précédentes, présentant la caractéristique supplémentaire suivante :
a. les nervures de butée (44) sont formées par plusieurs segments de nervures alignés les uns par rapport aux autres et de préférence respectivement disposés sur une flèche élastique (50).

8. Distributeur (10) selon l'une quelconque des revendications précédentes, présentant la caractéristique supplémentaire suivante :
a. l'unité d'évaluation électronique (40) comporte un boîtier électronique (41), et
b. la bande de fixation (60) flexible est conçue sous la forme d'un élément séparé et de préférence constitué d'un matériau différent de celui du boîtier électronique (41).

9. Distributeur (10) selon la revendication 8, présentant la caractéristique supplémentaire suivante :
a. la bande de fixation (60) est conçue sous la forme d'une bande élastique, présentant notamment de préférence au moins l'une des caractéristiques supplémentaires suivantes :
b. la bande de fixation (60) est conçue sous la forme d'un joint torique (61), et/ou
c. la bande de fixation (60) est conçue sous la forme d'une bande fermée qui entoure le boîtier sous la forme d'une double bande et qui est fixée à une unité principale de l'unité d'évaluation dans la zone de deux boucles de renvoi (60A), ou
d. la bande de fixation (60) est conçue sous la forme d'une bande fermée, qui est fixée au boîtier de manière à entourer complètement celui-ci.

10. Distributeur (10) selon l'une quelconque des revendications précédentes, présentant les caractéristiques supplémentaires suivantes :
a. la bande de fixation (60) comprend deux sections de bande (60B, 60C) qui sont respectivement fixées à une unité principale de l'unité d'évaluation (40) et qui sont reliées l'une à l'autre dans la zone d'une fermeture (62), ou
b. la bande de fixation (60) est conçue sous la forme d'une bande de longueur variable qui peut être réglée à une longueur souhaitée au moyen d'une fermeture (62),
de préférence avec l'une quelconque des caractéristiques supplémentaires suivantes :
c. la fermeture (62) est conçue sous la forme d'une fermeture à crochets et boucles, et/ou
d. la fermeture comporte des éléments de fermeture coopérants sur les deux sections de bande, une pluralité d'éléments de fermeture étant de préférence prévus sur l'une des sections de bande, et/ou
e. la fermeture (62) est conçue sous la forme d'une fermeture magnétique.

11. Distributeur (10) selon l'une quelconque des revendications précédentes, présentant les caractéristiques supplémentaires suivantes :
a. l'unité d'évaluation (40) comprend une unité de fixation (40A) et une unité électronique (40B), et
b. l'unité de fixation (40A) est conçue pour être fixée de manière amovible au boîtier (12), et
c. l'unité électronique (40B) est prévue pour être fixée de manière amovible à l'unité de fixation (40A),

12. Distributeur (10) selon la revendication 11, présentant au moins l'une des caractéristiques supplémentaires suivantes :
a. l'unité de fixation (40A) est conçue pour être fixée de manière amovible au boîtier (12) sans outils, et/ou
b. l'unité électronique (40B) est fixée magnétiquement à l'unité de fixation (40A), et/ou
c. l'unité électronique (40B) est fixée à l'unité de fixation (40A) au moyen d'un mécanisme d'encliquetage présentant une patte d'encliquetage (90) élastiquement déformable, et/ou
d. l'unité de fixation (40A) présente une caractéristique d'identification, en particulier stockée dans une puce RFID de l'unité de fixation, qui peut être lue par un capteur (26B) de l'unité électronique (40B).

13. Distributeur (10) selon l'une quelconque des revendications précédentes, présentant au moins l'une des autres caractéristiques suivantes :
a. l'unité d'évaluation (40) comprend au moins un capteur (26A, 26B, 26C), en particulier un capteur de mouvement (26A) et/ou un microphone (26C), et/ou
b. l'unité d'évaluation (40) comprend un dispositif de sortie (24), notamment sous la forme d'au moins une DEL, d'un dispositif d'affichage (24), d'un vibreur et/ou d'un haut-parleur,
c. l'unité d'évaluation comprend un module radio (22), en particulier un module radio Bluetooth et/ou WIFI (22), et/ou
d. l'unité d'évaluation comprend une source d'énergie (25), en particulier sous la forme d'une batterie.

14. Distributeur (10) selon l'une quelconque des revendications précédentes, présentant la caractéristique supplémentaire suivante :
a. le distributeur (10) est conçu sous la forme d'un distributeur de liquide (10), le réservoir de milieu (14) étant conçu sous la forme d'un réservoir de liquide (14),
en particulier avec au moins une des caractéristiques supplémentaires suivantes :
b. le distributeur est conçu sous la forme d'un distributeur à flacon souple et comporte un corps de réservoir élastiquement déformable du réservoir de liquide, par la compression manuelle duquel du liquide peut être expulsé, ou
c. le distributeur (10) est conçu sous la forme d'un distributeur à pompe qui comporte un dispositif de pompage (30) actionnable manuellement qui achemine le liquide du réservoir de liquide (14) vers l'ouverture de sortie (16), et/ou
d. le distributeur (10) est conçu sous la forme d'un pulvérisateur, et/ou
e. le distributeur est conçu sous la forme d'un inhalateur, en particulier sous la forme d'un distributeur comprenant un récipient qui peut être comprimé pour distribuer une dose du liquide,
f. le distributeur est conçu sous la forme d'un distributeur de gouttes ayant en particulier une surface de formation de gouttes entourant l'ouverture d'évacuation, et/ou
g. le distributeur est conçu sous la forme d'un distributeur nasal, comprenant en particulier un applicateur nasal allongé, à l'extrémité distale duquel est prévue l'ouverture de sortie, et/ou
h. le réservoir de milieu (14) du distributeur possède un volume d'au plus 200 ml, de préférence d'au plus 100 ml, et/ou
i. le réservoir de milieu (14) du distributeur est rempli d'un liquide pharmaceutique.

15. Unité d'évaluation pour un distributeur selon l'une quelconque des revendications précédentes, présentant les caractéristiques suivantes :
a. l'unité d'évaluation (40) est conçue pour être fixée de manière amovible à un distributeur (10), et
b. l'unité électronique d'évaluation (40) est conçue pour détecter l'utilisation du distributeur et comporte à cet effet au moins un capteur (26A, 26C), et
c. l'unité d'évaluation électronique (40) présente une face de butée (42) qui, lorsque l'unité d'évaluation (40) est montée, est orientée en direction d'un boîtier (12) du distributeur (10), et
d. l'unité électronique d'évaluation (40) présente, sur la face de butée (42), deux nervures de butée (44) parallèles l'une à l'autre destinées à venir en butée contre le boîtier (12), notamment pour être en butée contre un corps de réservoir (18) d'un réservoir de milieu (14) du distributeur (10), les nervures de butée (44) étant à une distance (46A) les unes des autres qui ne dépasse pas 80% du diamètre du corps de réservoir (18),
**caractérisé par** la particularité suivante :
e. l'unité d'évaluation électronique (40) comporte une bande de fixation (60) flexible, qui est fixée à l'unité d'évaluation (40) lors de l'utilisation et entoure le boîtier (12), en particulier le corps de réservoir (18),
de préférence avec au moins une des caractéristiques supplémentaires suivantes :
f. l'unité d'évaluation électronique (40) comprend une pluralité de bandes de fixation, qui peuvent facultativement être fixées à l'unité d'évaluation (40), en particulier à l'unité de fixation (40A), et/ou
g. l'unité d'évaluation (40) comprend une pluralité d'unités de fixation (40A) qui peuvent facultativement être fixées à l'unité électronique (40B).
